# EUROPEAN PATENT APPLICATION

(11) **EP 2 402 029 A1**
(43) Date of publication of application: **04.01.2012**
(21) Application number: 10305719.6
(22) Date of filing: 01.07.2010
(51) Int. Cl.: A61K 39/125, C07K 14/085, A61P 31/14

(54) **Enterovirus vaccines for preventing or treating type 1 diabetes (III)**

(71) Applicant: Sanofi Pasteur, 69007 Lyon Cedex (FR); Vactech OY, 33520 Tampere (FI)
(72) Inventor: Hyöty, Heikki, 33230, TAMPERE (FI); Knip, Mikael, 00130, HELSINKY (FI); Laitinen, Olli, 33270, TAMPERE (FI); Tolonen, Outi, 90120, OULU (FI); Pulkki, Minna, 33560, TAMPERE (FI); Oikarinen, Sami, 33500, TAMPERE (FI); Honkanen, Hanna-Riikka, 33520, TAMPERE (FI); Lecouturier, Valérie, 69380, CHAZAY d'AZERGUES (FR); Almond, Jeffrey, 69570, DARDILLY (FR)
(74) Representative: Boije-Backman, Solveig Magdalena

(57) **Abstract**

Coxsackie B virus CBV1 has been found to be strongly associated with the risk of contracting type 1 diabetes. A vaccine comprising CBV1, a component thereof or an antibody thereto is provided for use in preventing or treating type 1 diabetes.

## Description

### Field of the Invention

The present invention relates to a vaccine for preventing or treating type 1 diabetes. Especially the invention relates to a vaccine comprising an enterovirus, a component thereof or an antibody thereto. Said virus was found to be very strongly associated with the risk of contracting type 1 diabetes.

### Background of the Invention

Type 1 diabetes (T1 D) is a severe disease that has become more and more frequent already at a very early age. In type 1 diabetes the beta-cells of the pancreas are destroyed, which leads to insulin deficiency. The destruction of the beta-cells is believed to be caused by an autoimmune response, which in turn is assumed to be induced by a virus infection.

The connection between enteroviruses and type 1 diabetes has been documented in a multitude of studies. Enteroviruses have been detected in the pancreas, blood and intestinal mucosa of patients with type 1 diabetes more frequently than in control subjects and prospective studies have supported their role in the initiation of the beta-cell damaging process associated with type 1 diabetes. Enteroviruses infect pancreatic beta-cells in cell culture and cause diabetes in animal models. Enterovirus vaccines have therefore been suggested for preventing type 1 diabetes. However, the knowledge of which enterovirus serotypes that are involved in the disease is limited.

The group of enteroviruses includes more than 100 different serotypes, and because a vaccine covering all the 100 enterovirus serotypes is not realistic using the current standard vaccine technologies, the knowledge of which serotypes are involved in type 1 diabetes is critical for vaccine development. Enterovirus infections are usually subclinical, but they may also cause various kinds of diseases. For example polioviruses, coxsackie B virus (CBV), coxsackie A virus (CAV), and echovirus as well as numbered enteroviruses are enteroviruses known to be involved in the development of a variety of diseases. The spectrum of responsible serotypes varies a lot from disease to disease, and even in one disease like type 1 diabetes the exact serotypes have not been fully and reliably identified. Indeed, previous studies reporting association between enteroviruses and T1D were not able to discriminate between serotype or were restricted to case reports. Some studies have suggested that CBV serotypes may be important (Yoon et al, 1979 New Eng J of Med, v300, p1173; Hindersson M, et al., 2005, J Clin Virol v.33 p158; Dotta F. et al., 2007, PNAS 104: 5115). However, other enterovirus serotypes have also been sporadically reported (Cabrera-Rode et al., 2003, Diabetologia; Williams et al., 2006, J Clin Micro v.44 p441).

WO01/00236 (Hyöty et al.) suggests the use of oral poliovirus in a vaccine against type 1 diabetes. In addition it suggests another diabetes vaccine comprising one or more inactivated non-polio enteroviruses selected from the group consisting of CBV serotypes 1, 2, 3, 4, 5 and 6, echovirus serotypes 3, 4, 6, 9, 11, 22 and 30, CAV serotypes 9 and 16. However, no data is given as to the role of the individual serotypes listed, except for some animal tests with CBV3. A more recent publication US2010/0047273 (Rappuoli et al.) suggests the use of CBV4, and in particular of a Tuscany strain thereof for preventing or treating type 1 diabetes.

Still there is a great need for developing effective means and methods for preventing and treating type 1 diabetes. The present invention meets this need.

### Summary of the Invention

The present invention is based on the surprising finding that one enterovirus i.e. CBV1 is significantly more strongly associated with the risk of contracting type 1 diabetes than any other enterovirus serotype. For the first time the association between infection by individual enterovirus serotypes and T1D occurence has been demonstrated in humans, on a large case-control prospective study. This finding opens up opportunities for developing vaccines against type 1 diabetes using standard and well-known technologies used for example for poliovirus vaccines. The inventors estimate that a vaccine based on CBV1 alone would prevent about 50% of type 1 diabetes cases in at-risk children. It is striking that among all the serotypes studied, the ones which showed the most divergent association with type 1 diabetes belonged to the CBV group. This is even more striking when the low number of CBV serotypes is taken into account. The CBV group consists of six serotypes: 1, 2, 3, 4, 5 and 6, which are all structurally closely related. Still they have a very different influence on the induction of type 1 diabetes.

The present inventors found that children who had been in contact with CBV1 had an increased risk of contracting type 1 diabetes, while others who had been in contact with CBV3 or CBV6 had a reduced risk. This finding of the division of enteroviruses into protective and risk serotypes opens new therapeutic implications in the prevention and treatment of type 1 diabetes.

The present invention provides a vaccine comprising coxsackie B virus CBV1, a component thereof or an antibody thereto.

Further the invention provides this vaccine for use in preventing or treating type 1 diabetes.

A method of preventing or treating type 1 diabetes in a subject in need thereof comprising vaccinating the subject with a vaccine comprising coxsackie B virus CBV1, a component thereof or an antibody thereto is also disclosed.

Some preferred embodiment of the invention are set forth in the dependent claims

### Brief Description of the Drawings

Figure 1 shows the OR and CI values, and the prevalence of antibodies for the CBV serotypes.
Figure 2 shows a summary of the tight and loose criteria CBV1 time window analyses.
Figure 3 shows a combined population attributable risk (PAR) for CBV1 and CBV2.

### Detailed Description of the Invention

The present invention provides for the first time very strong evidence between the association of particular enterovirus serotypes and type 1 diabetes. More precisely serotype CBV1 was significantly associated with the risk of contracting type 1 diabetes. Furthermore, serotype CBV2 was also found to be at-risk for T1D. By contrast, serotypes CBV3 and CBV6 were found to be associated with a reduced risk for T1 D.

The disclosed vaccine comprises at least coxsackie B virus CBV1, a component thereof, or an antibody thereto in a pharmaceutically effective amount. The vaccine may comprise the virus in live, attenuated form, but conveniently it is inactivated. The vaccines may be used for preventing the development of T1 D or for alleviating or treating the disease.

A "pharmaceutically effective amount" of the vaccine is an amount, which is able to elicit an immune response that protects the vaccine recipient against the enterovirus either by eliciting neutralizing antibodies or a cell-mediated response, or both. In case of antibodies a pharmaceutical effective amount is one that mediates a protective immune response against the virus.

In case the vaccine comprises a component of the virus, the "component" may be an immunogenic structure of the virus such as a subunit thereof, or a nucleic acid fragment such as part of the genome of the virus. The component may also be recombinantly or synthetically produced or modified. Thus the vaccine may include whole viruses, the infectivity of which has been inactivated, or subunit vaccines containing certain antigenic structures, proteins or peptides of the virus, or their combination (such as virus like particles), or fragments of viral RNA or cDNA encoding the whole virus or individual viral proteins or inactivated forms of the virus. Inactivated vaccines may be produced by propagating the virus in cell cultures and by purifying it from infected cells and culture media by high-speed centrifugation in a density gradient formed by sucrose or other high-density media. Alternatively the virus may be purified by chromatography. The infectivity of the purified viruses is destroyed by inactivating the viruses by chemical treatment (e.g. formalin inactivation like that used to produce inactivated polio virus vaccine), irradiation or heat treatment. Subunit vaccines may consist of purified viral proteins or recombinant viral proteins, synthetic peptides corresponding to viral antigenic epitopes, virus like particles or empty viral capsids, which are produced during infection but lack the viral genome. These subunit vaccines can be administered either as such or conjugated to haptens or carriers (e.g. ISCOM particles, chitosan, TLR agonists, biodegradable microparticles).

In case the vaccine comprises an antibody against at least one of said enteroviruses, the antibody may be raised against the whole virus or against an immunogenic component thereof, or it may be recombinantly produced. The term antibody as used herein includes fragments of antibodies such as Fab-fragments and scFv fragments.

The above mentioned vaccines can be given parenterally by injections, perorally, intradermally, transcutaneously, sublingually, intranasally, as inhalation, or per rectum. Each immunizing dose includes viral structures in a titer, which is able to induce proper immune response in humans. This dose would correspond to that used in Salk-type inactivated poliovirus vaccine including 1.8 - 2 µg of viral protein per each dose and 20 - 40 antigenic D-units of poliovirus type 1,4 - 8 antigenic D-units of poliovirus type 2 and 16 - 32 antigenic D-units of poliovirus type 3. The dose may also be another, if it has been confirmed to be safe and immunogenic or able to stimulate the immune system.

Attenuated viruses are viruses of which the virulence has been reduced. This may be carried out by different methods including serial passage of the virus in cell cultures, antigenic modification by chemical treatments, construction of recombinant or chimeric viruses, mutagenization of viral genome, deletion or insertion of certain gene regions, selection of temperature sensitive mutants or irradiation. Alternatively, the enteroviruses may be attenuated natural virus isolates or infectious virus cDNA or RNA having reduced capability to cause clinical disease.

The live attenuated is conveniently formulated into a mucosal vaccine composition, which may be given perorally, sublingually, intranasally, as inhalation, or per rectum. Usually it is administered orally. Each immunizing dose includes infective viruses or infective RNA or cDNA in a titer, which is able to produce infection or activation of the innate or adaptive immune system or induce regulatory T-cells or regulatory cytokines in humans. This dose would correspond to that which is used in the traditional Sabin-type live oral poliovirus vaccine including a minimum of 10^{5.5} - 10⁶ TCID₅₀ for poliovirus Type 1, 10⁵ TCID₅₀ for poliovirus type 2 and 10^{5.5} - 10^{5.8} TCID₅₀ for poliovirus type 3 live attenuated Sabin strains of polioviruses. The dose may also be another, if it has been confirmed to be safe and infectious or able to activate the innate or adaptive immune system. (TCID = tissue culture infectious dose; TCID₅₀= the dose which infects 50% of the cultures.)

According to one embodiment of the invention, the CBV1 vaccine further comprises CBV2, a component thereof, or an antibody thereto. In still another embodiment the vaccine further comprises at least one additional diabetogenic enterovirus, a component thereof, or an antibody thereto. Like CBV1 also the optional other diabetogenic viruses are conveniently administered in inactivated form, as virus like particles or as recombinantly produced viral proteins.

The enteroviruses, their components or antibodies can be given in different combinations including one or more enterovirus serotypes given simultaneously or serially. If the different enteroviruses, their components or antibodies are given simultaneously the pharmaceutical composition may be in the form of a mixture of different enteroviruses, components or antibodies.

In a preferred embodiment of the invention the vaccine does not contain neither serotype CBV3 nor CBV6, or their components or antibodies. These serotypes should be excluded from the vaccine at least in the form that induces or mediates neutralizing antibodies such as inactivated viruses, their components or antibodies, so as to avoid elimination of the protective serotypes by the vaccine in the recipient.

The enterovirus, component or antibody is formulated into a pharmaceutical composition, which in addition to the active ingredients that elicit immune stimulation may comprise pharmaceutically acceptable excipients, carriers, haptens and adjuvants. Excipients, carriers, haptens and adjuvants may include for example phenoxyethanol, magnesium chloride, sucrose, thiomersal, formaldehyde, phenol, antibiotics (preservatives) or aluminium salts, polymer microparticles, ISCOM particles, carrier proteins (e.g. cholera toxin), liposomes, protein micelles (haptens/adjuvants), or TLR agonists.

The pharmaceutical composition is preferably administered to a child within 5 years after birth, and more preferably within 3 years, especially within 2 years, and most preferably within 1 year after birth, with boosters given later in life to prevent or treat type 1 diabetes. It can for example be given at the age or 3, 6 or 12 months, with boosters at older age. It can also be given to pregnant mothers to prevent type-1-diabetes in the baby, or prenatally to the pregnant mother and postnatally to the baby. When given to pregnant mothers the induced antibody response protects the child because IgG class maternal antibodies are transferred to the fetus through the placenta and are thus protecting the child until the age of 6 - 12 months when maternal antibodies disappear from the child's circulation.

The vaccination regime can be used in the whole population or in subpopulations carrying increased risk for type 1 diabetes. Such high-risk groups may include mothers or children with HLA-conferred disease susceptibility to type 1 diabetes, especially carriers of the HLA DR3 and/or DR4 alleles, subjects with type 1 diabetes in first or second-degree relatives or children testing positive for two or more diabetes-associated autoantibodies.

The vaccines described may be used in preventing and treating type 1 diabetes, in inducing an immune response against a diabetogenic enterovirus, and in eliminating the diabetogenic effect of an enterovirus in a subject in need thereof by vaccinating the subject with at least CBV1, a component thereof, or an antibody thereto.

### Example

### Seroneutralization analyses

Neutralizing antibodies were analyzed against a wide panel of different enterovirus serotypes in the same serum sample which was the first autoantibody positive sample taken during the prospective follow-up. Thus, this time point represents the initiation of the beta-cell damaging process. In addition, antibodies against those serotypes which were found to be interesting in this initial screening were measured at additional time-points to study the timing of infections and their relationship with the initiation of the beta-cell damaging process.

Altogether, seroneutralization analyses have been performed using 42 viruses and 522 serum/plasma samples (174 triplets, two control children for each case child). The completeness of the analyses in this virus set varies from 100% to 84% (Echo30) being for a majority of the viruses (35/40) more than 97.7%. In order to study the effect of the strain variation, two serotypes (CBV4 and Echo3) have been analyzed using both the freshly isolated wild type strains (wt) and corresponding reference (ATCC) strains (rs).

The viruses were analyzed in seroneutralization analyses using autoantibody seroconversion date samples. The viruses were CAV4, CAV5, CAV10, CAV16, EV71, CAV9, CBV1, CBV2, CBV3, CBV4-wt, CBV4-rs, CBV5, CBV6, Echo1, Echo2, Echo3-wt, Echo3-rs, Echo4, Echo5, Echo6, Echo7, Echo9, Echo11, Echo12, Echo13, Echo14, Echo15, Echo17, Echo18, Echo19, Echo20, Echo21, Echo25, Echo26, Echo27, Echo29, Echo30, Echo32, Echo33, EV74, EV78 and EV94.

The seroneutralization analyses were mainly carried out using a plaque neutralization assay. In this analysis the ability of the serum/plasma to inhibit a certain virus's ability to form plaques in cell layers has been determined as compared to controls, in which fetal calf serum has been used instead of human serum. In the analysis the inhibition has been considered to be significant (positive result, ++) when it has been more than 85 %. The inhibition range between 85-75% has been considered as weaker positive (+) and inhibition of less than 75% has been judged as a negative result. Because these analyses have been performed using two different serum/plasma dilutions (1/4 and 1/16) the results have been combined using the classification shown below. A minority of the analyses (such serotypes that did not form plaques) has been carried out using microneutralization analysis, in which the ability of the virus to kill cells can be monitored by CPE and via crystal violet vital staining. Similar classification was used for the results of microneutralization assay as for plaque inhibition assay.

| **Class** | **1:4** | **1:16** |
|---|---|---|
| Max positive (0) | ++ | ++ |
| Highly positive (1) | ++ | + |
| Moderately positive (2) | ++ | - |
| Positive (3) | + | - |
| Negative (4) | - | - |

### Seroneutralization results

The raw data was exported to Stata package and analyzed using conditional logistic regression models to evaluate the risk of certain serotypes to cause T1 D. The results of the conditional logistic regression analysis are given as odds ratios (ORs). If the OR in certain analysis is higher than 1 and the lower limit of the confidence interval (CI at the level of 95%) remains above 1 the virus can be considered as conferring risk for T1 D. On the other hand, if both the OR and the higher limit of the 95% CI are below 1, such a serotype can be considered as protective against T1 D. In such cases where OR is either over or below 1 and also the 95% CI includes values on both side of 1, the result is not statistically significant (P>0.05). In Table 1 the OR and CI values for the most interesting viruses are presented.

**Table 1. The OR and CI values of the most interesting serotypes. The statistically significant results are bolded**

| Virus | [OR (CI)] | [OR (Cl)] | [OR (Cl)] | |
|---|---|---|---|---|
| | 0-3 vs. 4 | 0-1 vs. 2-4 | 0-2 vs. 4 | 0-1 vs. 4 |
| CBV1 | **1.50 (1.02-2.23)** | 1.10 (0.65-1.87) | 1.56 | 1.39 |
| | | | (0.94-2.58) | (0.68-2.85) |
| CBV3 | **0.39 (0.18-0.82)** | 0.56 (0.24-1.34) | **0.36** | 0.50 |
| | | | **(0.15-0.85)** | (021-1.20) |
| CBV6 | **0.64 (0.41-0.97)** | 0.57 (0.21-1.59) | 0.86 | 049 |
| | | | (050-1.51) | (0.16-1.57) |

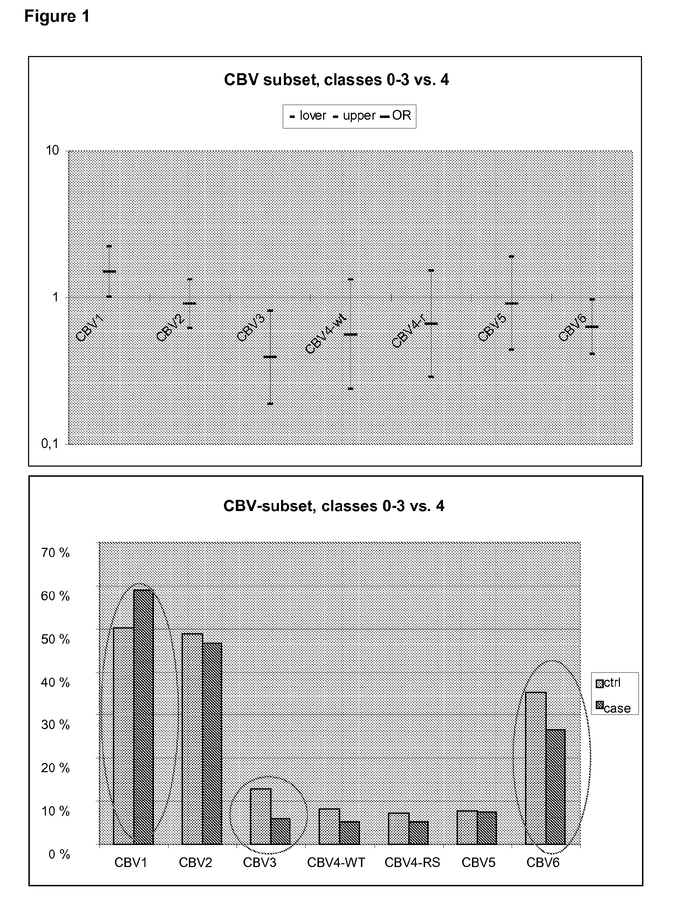

The prevalence of antibodies (percentage of children having neutralizing antibodies against each virus serotype) was also calculated. Figure 1 shows the results of the CBV-subset. The data represent the whole cohort. Classes 0-3 vs. 4, ORs are shown in the upper panel, and seroprevalence in the lower panel. Statistically significant results are marked with circles.

It was found that CBV1 is a clear risk serotype for type 1 diabetes while CBV3 and CBV6 are protective serotypes.. These associations were first drawn from seroneutralization results of a single cross-sectional time-point representing the first autoantibody positive sample, and were later confirmed in multi-time point analyses in which the timing of infections was analyzed in more detail. In these multi-time-point analyses the clearest result was the strong association of the CBV1 risk effect to the six month time window immediately preceding the first detection of type 1 diabetes related autoantibodies.

### Multi-time point seroneutralization analysis with CBV1, CBV3 and CBV6

To study the temporality of CBV1 infection with respect to AAB appearance, the following time points were selected for the seroneutralization analyses:
- 12 months before autoantibody seroconversion
- 6 months before autoantibody seroconversion
- point (first detection of autoantibodies; these samples were analyzed previously)
- 12 months after autoantibody seroconversion
- Time of the diagnosis of type 1 diabetes

According to this plan approximately 1 250 new samples fulfilling the criteria above were identified, collected, anonymized and tested.

All samples were screened using 1/4 and 1/16 serum dilutions. In this report the class comparison 03- vs. 4 and sensitivity analyses 1 and 2 has been generally utilized, except in the tight criteria analyses (explained below) in which infections were diagnosed by subjective judgments done by two independent researchers on the basis of pre-fixed criteria listed below for acute infection:

The acute infection was diagnosed according to following criteria, which had to be true for a classified infection:
- A seroconversion from titer 0 to titer 4 or higher
- The titer is 16 in at least one of the following samples
- All follow-up samples remain positive

All analyses were done blindly without knowing the case-control status of the child.

### Multi-time point statistical analyses

All statistical analyses presented are based on conditional logistic regression analyses. Three types of analyses were carried out for this data set. The timing of infections was determined and the time-relationship between infections and appearance of autoantibodies was analyzed. In other words, the frequency of infections among case children were compared to that in control children in each time window separately. The new time point results were analyzed similarly as described above by comparing cases and controls in each time point separately. These analyses were carried out in the whole group and in different subgroups according to the following list:
- Total data set from the whole cohort
- Gender
- Age
- HLA genotype
- Residence area
- Combinations of different AABs in certain follow-up samples
- Diagnosis of diabetes
- Cumulative protective effect

Seroneutralization analyses of these three viruses have been carried out using a plaque neutralization assay as described above.

Statistical analyses in loose and middle criteria approaches were done according to combined classes 0-3 vs. 4 and also sensitivity analyses, in which class 3 (sensitivity analysis 1) or both classes 2 and 3 (sensitivity analysis 2) are removed from the data set, were utilized. In the tight criteria approach the sensitivity analyses were not done due to its intrinsic "sensitivity" character.

### Timing of the infections

The timing of infections caused by CBV1 and the other five related serotypes was approached in the following way: First, the recognized infections were categorized to different time windows relative to the date of seroconversion to autoantibody positivity (AAB+ date). The used windows were as follows:
0. No infections or infection after the AAB+ date
1. Infection more than 12 months before the AAB+ date
2. Infection between 12 and 6 months before the AAB+ date
3. Infection within 6 months before the AAB+ date

In the loose criteria approach the first positive result was accepted as an infection regardless of the possibility of maternal antibodies or the possibility that results of the later time point samples turn to negative. The middle criteria approach was done similarly, except that those results that were biased by the identified maternal antibodies were nullified. This judgement were done by two independent experts who evaluated the data carefully and discarded those results in which the maternal antibodies could be the cause of the positivity. In the tight criteria approach the similar judgement procedure were applied. In short the acute infection according to tight criteria were as follows:
- a seroconversion from titer 0 to titer 4 or higher
- the titer is 16 in at least one of the following samples
- all follow-up samples remain positive

In addition to using these time-windows separately, analyses were also performed by combining some of these windows together.

Data is shown for the two serotypes (CBV1 and CBV2) identified to be at-risk. The timing of infections caused by the CBV1 risk serotype in these time windows is summarized in Tables 2 and 3. These analyses were done using the loose criteria and middle criteria and the risk effect of infection was analyzed by comparing its frequency in different time-windows to that in window zero (see above). The basic statistical analyses i.e. classes 0-3 vs. 4. and sensitivity analyses 1 and 2 were done.

**Table 2. CBV1 timing conditional logistic regression analysis, other windows vs. window 0. Loose criteria approach with classes 0-3 vs. 4 and sensitivity analyses 1 and 2.**

| **CBV1** | **Odds ratio** | **P-value** | **95% Conf. Interval** | |
|---|---|---|---|---|
| **Class 0-3 vs. 4** | | | | |
| Over 12 months before AAB+ date | 1.77 | 0.064 | 0.97 | 3.25 |
| 12-6 months before AAB+ date | **2.24** | **0.007** | **1.25** | **4.00** |
| 6-0 months before AAB+ date | **3.56** | **< 0.001** | **1.92** | **6.62** |

| **Sensitivity 1, Class 0-2 vs. 4** | | | | |
|---|---|---|---|---|
| Over 12 months before AAB+ date | 0.89 | 0.724 | 0.46 | 1.72 |
| 12-6 months before AAB+ date | 1.25 | 0.468 | 0.68 | 2.31 |
| 6-0 months before AAB+ date | **2.19** | **0.018** | **1.15** | **4.17** |

| **Sensitivity 2, Class 0-1 vs. 4** | | | | |
|---|---|---|---|---|
| Over 12 months before AAB+ date | 1.19 | 0.684 | 0.51 | 2.79 |
| 12-6 months before AAB+ date | 0.98 | 0.950 | 0.47 | 2.03 |
| 6-0 months before AAB+ date | 2.09 | 0.067 | 0.95 | 4.59 |

**Table 3. CBV1 timing conditional logistic regression analysis, other windows vs. window 0. Middle criteria approach with classes 0-3 vs. 4 and sensitivity analyses 1 and 2.**

| **CBV1** | **Odds ratio** | **P-value** | **95% Conf. Interval** | |
|---|---|---|---|---|
| **Class 0-3 vs. 4** | | | | |
| Over 12 months before AAB+ date | 1.62 | 0.262 | 0.70 | 3.75 |
| 12-6 months before AAB+ date | 0.77 | 0.417 | 0.41 | 1.44 |
| 6-0 months before AAB+ date | **2.09** | **0.002** | **1.32** | **3.33** |

| **Sensitivity 1, Class 0-2 vs. 4** | | | | |
|---|---|---|---|---|
| Over 12 months before AAB+ date | 1.14 | 0.795 | 0.41 | 3.17 |
| 12-6 months before AAB+ date | 0.66 | 0.307 | 0.30 | 1.46 |
| 6-0 months before AAB+ date | **2.10** | **0.007** | **1.23** | **3.57** |

| **Sensitivity 2, Class 0-1 vs. 4** | | | | |
|---|---|---|---|---|
| Over 12 months before AAB+ date | 0.70 | 0.629 | 0.16 | 3.02 |
| 12-6 months before AAB+ date | 0.36 | 0.078 | 0.11 | 1.12 |
| 6-0 months before AAB+ date | 1.44 | 0.342 | 0.68 | 3.05 |

Interestingly, in these analyses the CBV1 infections are clearly found more often in case children according to risk hypothesis with infections occurring close to the time of autoantibody seroconversion (at the time window 6-0 months before the AAB+ date). The result is same in all analyses, except that in sensitivity analysis 2 the statistical significance is missed. Because in this analysis we have accepted all positive antibody results (probably including false positive findings), except maternal antibodies in middle criteria analyses, it is essential to compare these results to those obtained using tight criteria for infections (Table 4).

**Table 4. CBV1 timing conditional logistic regression analysis, other windows vs. window 0. Tight criteria approach.**

| **CBV1** | **Odds ratio** | **P-value** | **95% Conf. Interval** | |
|---|---|---|---|---|
| Over 12 months before AAB+ date | 0.67 | 0.455 | 0.24 | 1.89 |
| 12-6 months before AAB+ date | 1.38 | 0.491 | 0.55 | 3.46 |
| 6-0 months before AAB+ date | **3.76** | **0.001** | **1.68** | **8.44** |

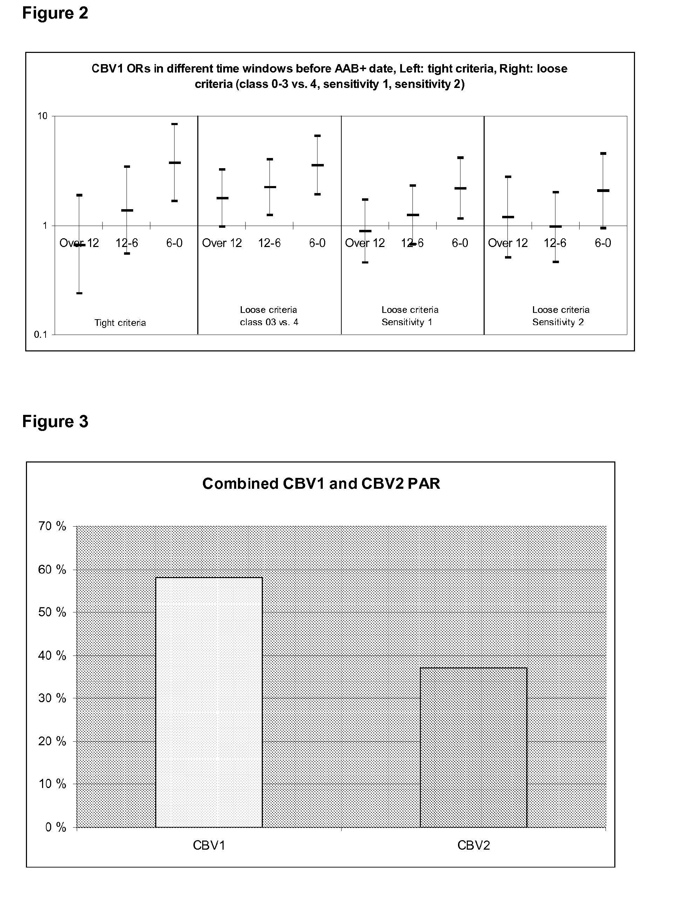

In this analysis, a lot of data have been removed to exclude all possible wrong positives. The results show that a credible dose response curve is observed highlighting the importance of the time window 6-0 months before the AAB+ date and proving a definite risk effect of CBV1 with convincing statistical outcome. Most importantly, because the same 6-0 months before the AAB+ date window was observed to be statistically significant in both the loose, middle and tight criteria approaches, these results cement this time period as a critical one for the CBV1 infection as a risk to induce T1 D. The tight criteria and loose criteria results are presented together in Figure 2, which shows CBV1 ORs in different time windows before AAB+ date, Left: tight criteria, Right: loose criteria (class 0-3 vs. 4, sensitivity 1, sensitivity 2). The dose response curves can be seen with both approaches being the most conspicuous in the tight criteria and loose criteria sensitivity 1 analyses.

In order to understand the significance of this result let us interpret it in the terms of the population attributable risk (PAR) which estimates the proportion of type 1 diabetes cases which could be prevented by the CBV1 vaccine in the population. Assuming that the OR is 3.76 (= tight criteria result for window 6-0 months before the AAB+ date) and the prevalence of the CBV1 infection is 50% as indicated by the prevalence of CBV1 antibodies in AAB+ time point in control subjects, the PAR equation results in 58% for CBV1 alone (Figure 3). Figure 3 shows the population attributable risk (PAR) for CBV1 calculated using an OR of 3.76 and a prevalence (Pc) of 50% representing the seroprevalence at the time of autoantibody seroconversion in control subjects.

In the following analysis the time windows 6-0 and 12-6 were combined. The risk effect of CBV1 was significant also in this combined time window analysis (Table 5).

**Table 5. CBV1 timing conditional logistic regression analysis, 12-0 window against window 0. Tight criteria approach.**

| **CBV1** | **Odds ratio** | **P-value** | **95% Conf. Interval** | |
|---|---|---|---|---|
| Over 12 months before AAB+ date | 0.66 | 0.434 | 0.24 | 1.85 |
| 12-0 months before AAB+ date | **2.46** | **0.004** | **1.33** | **4.53** |

Because the strongest risk effect was seen in the 6-0 window, this time period was analyzed using another type of comparison. Instead of comparing it against window 0 (which was done in the previous analyses), this window was compared to all other windows in combination. Again, the previous findings showing the critical importance of this time-window were supported by this analysis (Table 6).

**Table 6. CBV1 timing conditional logistic regression analysis, 6-0 window against combined other time windows. Tight criteria approach.**

| **CBV1** | **Odds ratio** | **P-value** | **95% Conf. Interval** | |
|---|---|---|---|---|
| 6-0 months before AAB+ date | **3.78** | **0.001** | **1.69** | **8.43** |

### Multi-time point seroneutralization analysis further including CBV2

In this analysis also CBV2 serotype was analyzed in similar multi-time-point setting as CBV1.

### Timing of infections

To clarify the possible timing of CBV subgroup infections, the same time points were selected for the seroneutralization analyses as described above.

The results presented below are based on the seroneutralization analysis of about 2 000 samples. In order to study the timing of infections caused by the six CBV serotypes the recognized infections were categorized to different time windows relative to the date of seroconversion to autoantibody positivity (AAB+ date). The used windows were numbered from 0 to 3 as described above.

The same loose, middle and tight criteria approaches were used as described above. Statistical analyses were done using conditional logistic regression. Since some additional laboratory results have been added to the results are shown here also for CBV1. The results of statistical analyses for the six CBV serotypes are shown in Tables 7-8.

**Table 7. CBV1 infection timing analysis. Loose criteria approach with classes 0-3 vs. 4.**

| **CBV1** | **Odds ratio** | **P-value** | **95% Conf. Interval** | |
|---|---|---|---|---|
| **Class 0-3 vs. 4** | | | | |
| Over 12 months before AAB+ date | **1.89** | **0.035** | **1.05** | **3.40** |
| 12-6 months before AAB+ date | **2.27** | **0.005** | **1.28** | **4.03** |
| 6-0 months before AAB+ date | **3.54** | **<0.001** | **1.95** | **6.42** |

**Table 8. CBV2 infection timing analysis. Loose, Middle and Tight criteria approaches with classes 0-3 vs. 4.**

| **CBV2** | **Odds ratio** | **P-value** | **95% Conf. Interval** | |
|---|---|---|---|---|
| **Loose criteria** | | | | |
| Over 12 months before AAB+ date | 0.79 | 0.441 | 0.44 | 1.43 |
| 12-6 months before AAB+ date | 1.38 | 0.236 | 0.81 | 2.34 |
| 6-0 months before AAB+ date | **2.32** | **0.001** | **1.40** | **3.84** |

| **Middle criteria** | | | | |
|---|---|---|---|---|
| Over 12 months before AAB+ date | 0.80 | 0.523 | 0.41 | 1.58 |
| 12-6 months before AAB+ date | 1.02 | 0.946 | 0.54 | 1.93 |
| 6-0 months before AAB+ date | **2.29** | **0.001** | **1.43** | **3.66** |

| **Tight Criteria** | | | | |
|---|---|---|---|---|
| Over 12 months before AAB+ date | 0.88 | 0.838 | 0.26 | 3.00 |
| 12-6 months before AAB+ date | 1.00 | 0.994 | 0.33 | 3.03 |
| 6-0 months before AAB+ date | **2.44** | **0.059** | **0.97** | **6.15** |

### Conclusions

In addition to CBV1 also the closely related CBV2 was found to be a risk virus in multi-time-point analyses especially in the 6-0 months before AAB+ time window. Thus, the multi-time-point analyses produced a good second candidate for the preventive vaccine cocktail without forgetting the protective viruses found earlier.

PAR was also calculated for CBV2 using loose criteria OR=2.26 and the seroprevalence of 48% in background population, accordingly, we end up with PAR=37% (Figure 3). The result indicates that a vaccine cocktail combination of CBV1 and CBV2 is most interesting. These two viruses can lead to a product having a major effect to prevent the onset of the type 1 diabetes.

Altogether, the results indicate that CBV2 is also a risk virus associated with the pathogenesis of T1 D and therefore useful in the development of the vaccine.

## Claims

1. A vaccine comprising coxsackie B virus CBV1, a component thereof or an antibody thereto.

2. The vaccine of claim 1, wherein the coxsackie B virus CBV1 is inactivated.

3. The vaccine of claim 1, further comprising coxsackie B virus CBV2, a component thereof or an antibody thereto.

4. The vaccine of claim 3, wherein the coxsackie B virus CBV2 is inactivated.

5. The vaccine of any one of claims 1 to 4, further comprising at least one additional diabetogenic enterovirus, a component thereof or an antibody thereto.

6. The vaccine of any one of claims 1 to 5, which does not comprise inactivated coxsackie virus CBV3 nor CBV6, a component thereof, or an antibody thereto.

7. The vaccine of claim 1 or 3, wherein the component is a subunit or a nucleic acid.

8. The vaccine of any one of claims 1 to 7 further comprising a pharmaceutically acceptable excipient and adjuvant.

9. The vaccine of any one of claims 1 to 8 for use in preventing or treating type 1 diabetes.

10. The vaccine of claim 9, wherein the use further comprises vaccinating with CBV2, a component thereof or an antibody thereto.

11. The vaccine of claim 9 or 10, wherein the use comprises administering it to a pregnant woman, and optionally postnatally to her baby.

12. Method of preventing or treating type 1 diabetes in a subject in need thereof comprising vaccinating the subject with a vaccine comprising coxsackie B virus CBV1, a component thereof or an antibody thereto.

13. The method of claim 12, further comprising vaccinating the subject with a vaccine comprising coxsackie B virus CBV2, a component thereof or an antibody thereto.
